# FASCICULE DE BREVET EUROPEEN

(11) **EP 0 603 080 B1**
(45) Date de publication et mention de la délivrance du brevet: **10.07.1996**
(21) Numéro de dépôt: 93403080.0
(22) Date de dépôt: 17.12.1993
(51) Int. Cl.: A61K 7/02, A61K 7/00, A61K 9/107

(54) **Composition cosmétique ou dermatologique biphase pour le démaquillage, le nettoyage ou le soin de la peau contenant un chlorure d'alkyldiméthylbenzylammonium**
Kosmetische oder dermatologische Zwei-Phasen-Zusammensetzungen zum Abschminken, zur Hautreinigung oder zur Hautpflege, enthaltend Alkyldimethylbenzylammoniumchlorid
Cosmetic or dermatologic two-phase composition for make-up removal, skin cleaning or skin care containing an alkyldimethylbenzylammonium chloride

(30) Priorité: 18.12.1992 FR 9215309
(43) Date de publication de la demande: 22.06.1994
(73) Titulaire: L'OREAL, F-75008 Paris (FR)
(72) Inventeur: Touzan, Philippe, F-31520 Ramonville-Saint-Agne (FR); Lukassen, Liliane, F-94550 Chevilly-Larue (FR); Louvet, Nathalie, F-94240 L'Hay-Les-Roses (FR)
(74) Mandataire: Nony, Michel

(56) Documents cités:
- EP-A- 0 046 594
- WO-A-90/10429
- GB-A- 2 071 495
- GB-A- 2 219 937
- DATABASE WPI Week 1379, Derwent Publications Ltd., London, GB; AN 79-25068B & JP-A-54 024 908 (KANEBO) 24 Février 1979
- DATABASE WPI Week 3086, Derwent Publications Ltd., London, GB; AN 86-194673 & JP-A-61 129 033 (SHISEIDO KK) 17 Juin 1986
- PATENT ABSTRACTS OF JAPAN vol. 59, no. 112 (C-441) & JP-A-62 059 204 (SHISEIDO CO LTD) 21 Aoüt 1987
- DATABASE WPI Week 3378, Derwent Publications Ltd., London, GB; AN 78-59815A & JP-A-53 080 460 (LION DENTIFRICE KK) 15 Juillet 1978

## Description

La présente invention a pour objet une composition cosmétique ou dermatologique pour le démaquillage, le nettoyage ou le soin de la peau constituée de deux phases distinctes, une phase aqueuse et une phase huileuse, s'émulsionnant facilement par agitation, mais se démixant rapidement au repos.

Les compositions de ce type constituées de deux phases distinctes notamment d'une phase aqueuse et d'une phase huileuse sont généralement désignées sous le terme de "composition biphase". L'utilisation desdites compositions nécessite une agitation préalable afin de former une émulsion, celle-ci devant être de qualité et de stabilité suffisantes pour permettre une application homogène des deux phases. Au repos, lesdites phases doivent se séparer rapidement et retrouver leur état initial, ce phénomène étant plus connu sous le terme de "déphasage".

Des compositions biphases ont déjà été décrites dans le brevet FR 88.14641 (2.638.636) notamment pour le démaquillage des yeux. Les compositions de ce brevet, bien qu'ayant d'excellentes propriétés démaquillantes présentent des problèmes de déphasage dans la mesure où, après agitation, elles ont tendance à rester à l'état émulsionné.

En raison des agitations successives, ce phénomène s'amplifie et les temps de déphasage peuvent atteindre alors plusieurs heures voire plusieurs jours.

De plus, ce phénomène est accru par la présence, en quantité importante, d'huiles lourdes et/ou d'agents tensio-actifs dans la composition, ce qui implique de réduire la quantité d'agent tensio-actif et à préférer, aux dépens des huiles lourdes, les huiles légères et volatiles.

Or l'obtention d'un déphasage rapide est souhaitable pour diverses raisons notamment parce qu'une mauvaise séparation des deux phases est perçue comme étant inesthétique par les utilisateurs.

En outre, dans ce type de composition biphase, il est généralement utile de pouvoir incorporer dans la phase huileuse des principes actifs liposolubles mais ceux-ci sont pour la plupart peu stables au contact d'une phase hydrophile. Parmi ces principes actifs lipophiles peu stables au contact d'une phase hydrophile, on peut citer notamment les principes actifs dermatologiques tels que des antifongiques comme l'Econazole et le Miconazole, des antibactériens comme le Chlorquinol, l'Hexachlorophène, l'acide usnique, des dérivés kératolytiques comme l'acide salicylique et des anti-inflammatoires comme l'γ-oryzanol et l'α-bisabolol. Afin de maintenir l'intégrité de ces principes actifs, il est donc particulièrement souhaitable de limiter leur temps de contact avec la phase hydrophile et, par conséquent, d'accélérer le processus de déphasage.

Après de nombreuses études, on a maintenant constaté de façon surprenante et inattendue qu'en associant un agent tensio-actif et un agent de déphasage particulier, on obtenait des compositions s'émulsionnant facilement par agitation et se démixant rapidement au repos.

La présente invention a pour objet une composition cosmétique ou dermatologique non moussante se présentant sous forme biphasique constituée d'une phase aqueuse et d'une phase huileuse distincte en un rapport compris entre 30/70 et 60/40, l'une au moins desdites phases contenant un agent tensio-actif, caractérisée par le fait qu'elle contient en outre dans ladite phase aqueuse un agent de déphasage en une proportion comprise entre 0,025 % et 5 % en poids, ledit agent étant un chlorure d'alkyldiméthylbenzylammonium de formule : 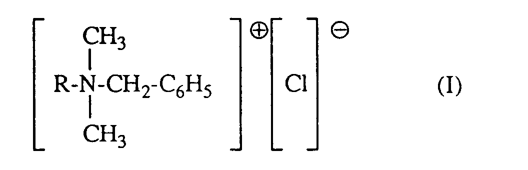
dans laquelle :
R représente un radical alkyle linéaire saturé ayant de 12 à 16 atomes de carbone, ou un mélange de chlorures d'alkyldiméthylbenzylammonium de formule (I), et que ledit agent tensio-actif est présent en une proportion supérieure à 0,25% en poids par rapport au poids de la composition, celui-ci étant du type anionique, non ionique ou amphotère lorsqu'il est présent dans la phase aqueuse ou du type non ionique liposoluble lorsqu'il est présent dans la phase huileuse.

De préférence, l'agent de déphasage utilisé dans la composition selon l'invention est un mélange de chlorures d'alkyldiméthylbenzylammonium de formule (I) constitué d'environ 65 % en poids de chlorure de lauryldiméthylbenzyl ammonium, d'environ 23% en poids de chlorure de myristyldiméthylbenzylammonium et d'environ 8 % en poids de chlorure de palmityldiméthylbenzylammonium, le reste étant constitué par au moins un chlorure d'alkyldiméthylammonium dont le radical a moins de 12 ou plus de 16 atomes de carbone.

Comme mélange de chlorures d'alkyldiméthylbenzylammonium utilisable selon l'invention, on peut citer celui commercialisé sous la dénomination de "chlorure de benzalkonium" par la Société Fluka, dont les caractéristiques sont les suivantes :
Poids moléculaire : 360
Point de fusion : 35°C
En vue de mettre en évidence les excellentes propriétés de déphasage des chlorures d'alkyldiméthylbenzylammonium utilisés selon l'invention, de nombreux essais ont été effectués sur différents ammonium quaternaires de structure voisine de celle des composés de formule (I).

Ainsi, les études effectuées avec les composés suivants : le chlorure d'oléyldiméthylbenzylammonium, le chlorure de distéaryldiméthylammonium, le chlorure de stéaryldiméthylbenzylammonium,le bromure de myristyltriméthylammonium, le chlorure de lauryltriméthylammonium, le bromure de lauryltriméthylammonium, le bromure de stéaryltriméthylammonium, le chlorure de myristalkonium et le bromure de cethexonium se sont avérées montrer qu'aucun de ceux-ci n'avaient une influence quelconque sur le déphasage. De façon tout à fait surprenante, seuls les chlorures d'alkyldiméthylbenzylammonium de formule (I) et leurs mélanges se sont avérés présenter cette propriété d'agent de déphasage. Ceci est d'autant plus étonnant que ces composés sont des molécules amphiphiles, connus de plus comme étant des tensio-actifs, et que, par conséquent, ils devraient stabiliser le mélange des phases aqueuse et huileuse au lieu de favoriser leur séparation.

Parmi les agents tensio-actifs anioniques pouvant être présents dans la phase aqueuse de la composition selon l'invention, on peut notamment citer :
- les alkyléthers sulfates tels que le produit vendu sous la dénomination de "Texapon ASV" par la Société Henkel,
- les alkylsulfoacétates tels que le produit vendu sous la dénomination de "Lathanol Lal" par la Société Stepan,
- les sulfosuccinates d'alkyle tels que le produit vendu sous la dénomination de "Sodium Dioctyl Sulfosuccinate" par la Société Rhone Poulenc,
- les alkylamidosulfosuccinates tels que le produit vendu sous la dénomination de "Rewoderm S 1333" par la Société Rewo,
- les alkylamidopolypeptides tels que le produit vendu sous la dénomination de "Lamepon S" par la Société Grunau, et
- les alkylsarcosinates tels que le produit vendu sous la dénomination de "Oramix L 30" par la Société Seppic.

Parmi les agents tensio-actifs amphotères pouvant être présents dans la phase aqueuse de la composition selon l'invention, on peut notamment citer :
- les alkylamidopropyldiméthylbétaïnes tels que le produit vendu sous la dénomination de "Tego Betaine L 7" par la Société Goldschmidt,
- les alkylamidobétaïnes tels que le produit vendu sous la dénomination de "Incronam 30" par la Société Croda,
- les dérivés d'imidazoline tels que le produit vendu sous la dénomination de "Chimexane HD" par la Société Chimex, et
- les N-(N'-acylaminoéthyl)N-hydroxyéthyl β-alaninates de sodium tels que le produit vendu sous la dénomination de "Monateric Isa 35" par la Société Mona.

Selon un mode de réalisation préféré de l'invention, l'agent tensio-actif, présent dans la phase aqueuse de la composition biphase, est du type non ionique.

Parmi les agents tensio-actifs non ioniques, ceux particulièrement préférés sont:
- les esters de sorbitol polyoxyéthylénés tels que le produit vendu sous la dénomination de "Tween 20" par la Société Atlas,
- les alcools gras polyoxyéthylénés tels que le produit vendu sous la dénomination de "Remcopal 21912 AL" par la Société Gerland,
- les alkylphénols polyoxyéthylénés tels que le produit vendu sous la dénomination de "Triton X 100" par la Société Rohm-Haas, et
- les condensats d'oxyde d'éthylène et d'oxyde de propylène tels que ceux vendus sous les dénominations de "Synperonic PE" par la Société ICI et en particulier ceux référencés L31, L64, F38, F88, L92, P103, F108 et F127,
- les polymères blocs polyoxyéthylène, polyoxypropylène tels que ceux vendus sous les dénominations "Poloxamer" ou "Pluronic" par la Société BASF.

Parmi les agents tensio-actifs non-ioniques liposolubles pouvant être présents dans la phase huileuse de la composition selon l'invention, on peut notamment citer:
- les esters d'acide gras, ayant 12 à 18 atomes de carbone, et de sorbitol comme le polysorbate 85 vendu sous la dénomination "Tween 85" par la Société ICI ou les "Arlacel" vendus également par la Société ICI,
- les esters d'acide gras polyglycérolés ayant 12 à 18 atomes de carbone, comme le dioléatate polyglycérolé vendu sous la dénomination "Decaglyn 2-0" par la Société Nikkol,
- les alcools gras oxyéthylénés et les alkylphénols oxyéthylénés, comme le nonoxynol 7 qui est un nonylphénol oxyéthyléné à 7 moles d'O.E. vendu sous la dénomination de "Synperonic NP7" par la Société ICI.

Selon un mode de réalisation préféré de l'invention, la proportion en agent tensio-actif présent dans l'une au moins desdites phases est comprise entre 0,5 % et 10 % en poids par rapport au poids total de la composition.

Le rapport entre l'agent tensio-actif et l'agent de déphasage est de préférence compris entre 0,1/1 et 200/1.

La phase aqueuse de la composition peut être constituée par de l'eau déminéralisée stérile ou par une eau florale telle que de l'eau de rose, de l'eau de bleuet, de l'eau de camomille ou de l'eau de tilleul.

La phase huileuse de la composition biphase selon l'invention est constituée d'un mélange d'huiles, celles-ci pouvant être des huiles minérales, végétales ou synthétiques ou encore des huiles de silicone.

Parmi les huiles minérales pouvant constituer la phase huileuse, on peut notamment citer l'huile de vaseline et les hydrocarbures aliphatiques supérieurs tels que par exemple l'isohexadécane ; parmi les huiles végétales, l'huile de jojoba ainsi que l'huile de carthame; parmi les huiles de silicone, le cyclopentadiméthylsiloxane vendu sous la dénomination de "Volatil Silicone 7158" par la Société Union Carbide et parmi les produits de synthèse, les palmitates d'alkyle ayant de 2 à 10 atomes de carbone tels que le palmitate d'isopropyle, ou le palmitate d'éthyl-2 hexyle et les adipates d'alkyle ayant de 2 à 10 atomes de carbone tels que l'adipate de diéthyl-2 hexyle.

Selon un mode de réalisation préféré de l'invention, la phase huileuse est constituée d'au moins une huile choisie parmi une cyclométhicone en une proportion de 1 à 80 %, de l'isohexadécane de 1 à 50 %, du palmitate d'octyle de 1 à 50 % et de l'adipate de dioctyle de 1 à 50 % en poids par rapport au poids total de la phase huileuse.

La composition biphase selon l'invention peut également contenir des adjuvants cosmétiques ou dermatologiques conventionnels présents dans l'une au moins des deux phases selon leur nature hydrophile ou lipophile tels que par exemple des parfums, des agents conservateurs, des colorants, des agents adoucissants, un tampon, des humectants et éventuellement un électrolyte tel que le chlorure de sodium pour apporter une isotonicité dans la phase aqueuse.

Parmi les agents humectants, on peut notamment mentionner la glycérine et les glycols tels que l'hexylèneglycol, le polyéthylèneglycol 600 et le polypropylèneglycol, ceux-ci étant présents à une concentration comprise entre 0,05 et 2 %.

Parmi les agents adoucissants, on peut en particulier citer l'allantoïne et certains extraits de plantes.

La composition biphase selon l'invention peut être utilisée à toute fin cosmétique ou dermatologique et notamment pour le démaquillage des yeux ou du visage, en lotion solaire ou en lotion pour le nettoyage ou le soin de la peau.

On va maintenant donner à titre d'illustration, plusieurs exemples de compositions cosmétiques de démaquillage, de nettoyage et de soin de la peau selon l'invention.

### EXEMPLE 1

Une lotion démaquillante biphase pour les yeux, selon l'invention, est obtenue en conditionnant dans un flacon 48 % d'une phase huileuse (*A*) et 52 % d'une phase aqueuse (*B*) contenant les ingrédients suivants :

| *A - Phase huileuse* | % |
|---|---|
| - Palmitate d'octyle | 30 |
| - Adipate de dioctyle | 15 |
| - Cyclométhicone | 55 |

| *B - Phase aqueuse* | % |
|---|---|
| - Polymère bloc polyoxyéthylène, polyoxypropylène vendu par la Société BASF sous la dénomination "Poloxamer 407" | 0,5 |
| - Phosphate dipotassique | 0,3 |
| - Phosphate monopotassique | 0,1 |
| - Chlorure de sodium | 0,9 |
| - Mélange de chlorures d'alkylméthylbenzylammonium commercialisé sous la dénomination de "chlorure de benzalkonium" par la Société Fluka | 0,1 |
| - Parfum | 0,0175 |
| - Conservateurs qs | |
| - Eau | qsp 100 |

Après agitation et usage, le déphasage total entre les deux phases intervient après environ 15 minutes à température ambiante.

### EXEMPLE 2

Une lotion adoucissante et hydratante biphase pour le corps, selon l'invention, est obtenue en conditionnant dans un flacon 50 % d'une phase huileuse (*A*) et 50 % d'une phase aqueuse (*B*) contenant les ingrédients suivants :

| *A - Phase huileuse* | % |
|---|---|
| - Cyclométhicone | 30 |
| - Isohexadécane | 20 |
| - Huile de vaseline | 47 |
| - Huile de jojoba | 3 |

| *B - Phase aqueuse* | % |
|---|---|
| - Mélange de chlorures d'alkylméthylbenzylammonium commercialisé sous la dénomination de "chlorure de benzalkonium" par la Société Fluka | 0,15 |
| - Polysorbate 20 vendu par la Société ICI sous la dénomination "Tween 20" | 2 |
| - Glycérine | 6 |
| - Conservateurs qs | |
| - Eau | qsp 100 |

### EXEMPLE 3

Une lotion solaire biphase, selon l'invention, est obtenue en conditionnant dans un flacon 70 % d'une phase huileuse (*A*) et 30 % d'une phase aqueuse (*B*) contenant les ingrédients suivants :

| *A - Phase huileuse* | % |
|---|---|
| - Palmitate d'octyle | 20 |
| - Isohexadécane | 50 |
| - Adipate de dioctyle | 25 |
| - p-méthoxycinnamate de 2-éthyl hexyle | 5 |

| *B - Phase aqueuse* | % |
|---|---|
| - Mélange de chlorures d'alkylméthylbenzylammonium commercialisé sous la dénomination de "chlorure de benzalkonium" par la Société Fluka | 0,20 |
| - Polymère bloc polyoxyéthylène, polyoxypropylène vendu par la Société BASF sous la dénomination "Poloxamer 184" | 1 |
| - Polypropylèneglycol | 3 |
| - Conservateurs qs | |
| - Eau | qsp 100 |

### EXEMPLE 4

Une lotion biphase, adoucissante et démaquillante, pour le visage, selon l'invention, est obtenue en conditionnant dans un flacon 45 % d'une phase huileuse (*A*) et 55 % d'une phase aqueuse (*B*) contenant les ingrédients suivants :

| *A - Phase huileuse* | % |
|---|---|
| - Isohexadécane | 39 |
| - Cyclométhicone | 60 |
| - Laurate de sorbitan vendu par la Société ICI sous la dénomination "Arlacel 20" | 1 |

| *B - Phase aqueuse* | % |
|---|---|
| - Phosphate monopotassique | 0,1 |
| - Phosphate dipotassique | 0,3 |
| - Mélange de chlorures d'alkylméthylbenzylammonium commercialisé sous la dénomination de "chlorure de benzalkonium" par la Société Fluka | 0,04 |
| - Eau de rose | 20 |
| - Colorant | 0,05 |
| - EDTA tétrasodique | 0,1 |
| - Conservateurs qs | |
| - Eau | qsp 100 |

## Revendications

1. Composition cosmétique ou dermatologique non moussante se présentant sous forme biphasique constituée d'une phase aqueuse et d'une phase huileuse distincte en un rapport compris entre 30/70 et 60/40, l'une au moins desdites phases contenant un agent tensio-actif, caractérisée par le fait qu'elle contient en outre dans ladite phase aqueuse un agent de déphasage en une proportion comprise entre 0,025 % et 5 % en poids, ledit agent étant un chlorure d'alkyldiméthylbenzylammonium de formule : 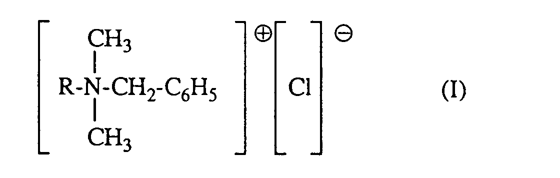 dans laquelle:
R représente un radical alkyle linéaire saturé ayant de 12 à 16 atomes de carbone, ou un mélange de chlorures d'alkyldiméthylbenzylammonium de formule (I), et que ledit agent tensio-actif est présent en une proportion supérieure à 0,25 % en poids par rapport au poids de la composition, celui-ci étant du type anionique, non ionique ou amphotère lorsqu'il est présent dans la phase aqueuse ou du type non ionique liposoluble lorsqu'il est présent dans la phase huileuse.

2. Composition selon la revendication 1, caractérisée par le fait que ledit agent de déphasage est un mélange constitué d'environ 65 % en poids de chlorure de lauryldiméthylbenzyl ammonium, d'environ 23% en poids de chlorure de myristyldiméthylbenzylammonium et d'environ 8 % en poids de chlorure de palmityldiméthylbenzylammonium, le reste étant constitué par au moins un chlorure d'alkyldiméthylammonium dont le radical a moins de 12 ou plus de 16 atomes de carbone.

3. Composition cosmétique ou dermatologique selon l'une quelconque des revendications précédentes, caractérisée par le fait que l'agent tensio-actif est présent dans l'une au moins des deux phases de la composition en une proportion comprise entre 0,5 et 10% en poids par rapport au poids total de la composition.

4. Composition cosmétique ou dermatologique selon l'une quelconque des revendications précédentes, caractérisée par le fait que le rapport entre l'agent tensio-actif et l'agent de déphasage est compris entre 0,1/1 et 200/1.

5. Composition cosmétique ou dermatologique selon l'une quelconque des revendications précédentes, caractérisée par le fait que la phase huileuse contient au moins une huile prise dans le groupe constitué par: l'huile de vaseline, l'isohexadecane, une huile de silicone, une huile synthétique ou une huile végétale choisie parmi l'huile de jojoba et l'huile de carthame.

6. Composition cosmétique ou dermatologique selon la revendication 5, caractérisée par le fait que l'huile synthétique est un palmitate d'alkyle ou un adipate d'alkyle, le radical alkyle ayant de 2 à 10 atomes de carbone.

7. Composition cosmétique ou dermatologique selon l'une quelconque des revendications précédentes, caractérisée par le fait que la phase huileuse est constituée d'au moins une huile choisie parmi une cyclométhicone en une proportion de 1 à 80 %, de l'isohexadécane de 1 à 50 %, du palmitate d'octyle de 1 à 50 % et de l'adipate de dioctyle en une proportion de 1 à 50 % en poids par rapport au poids total de la phase huileuse.

8. Composition cosmétique ou dermatologique selon l'une quelconque des revendications précédentes, caractérisée par le fait qu'elle contient en outre, dans au moins une des deux phases au moins un adjuvant cosmétique conventionnel pris dans le groupe constitué par: un parfum, un agent conservateur, un colorant, un agent adoucissant, un tampon, un humectant et un électrolyte.

## Claims

1. Cosmetic or dermatological non-foaming composition provided in two-phase form consisting of an aqueous phase and of a separate oily phase in a ratio between 30/70 and 60/40, at least one of the said phases containing a surface-active agent, characterized in that it additionally contains, in the said aqueous phase, a dephasing agent in a proportion between 0.025% and 5% by weight, the said agent being an alkyldimethylbenzylammonium chloride of formula: 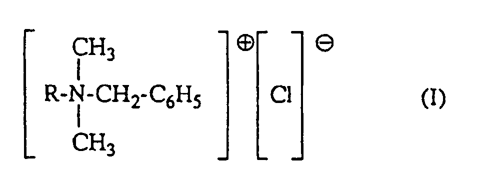 in which:
R represents a saturated linear alkyl radical having from 12 to 16 carbon atoms, or a mixture of alkyldimethylbenzylammonium chlorides of formula (I), and in that the said surface-active agent is present in a proportion greater than 0.25% by weight relative to the weight of the composition, the surface-active agent being of the anionic, nonionic or amphoteric type when it is present in the aqueous phase or of the liposoluble nonionic type when it is present in the oily phase.

2. Composition according to Claim 1, characterized in that the said dephasing agent is a mixture consisting of approximately 65% by weight of lauryldimethylbenzylammonium chloride, of approximately 23% by weight of myristyldimethylbenzylammonium chloride and of approximately 8% by weight of palmityldimethylbenzylammonium chloride, the remainder consisting of at least one alkyldimethylammonium chloride the radical of which has less than 12 or more than 16 carbon atoms.

3. Cosmetic or dermatological composition according to either of the preceding claims, characterized in that the surface-active agent is present in at least one of the two phases of the composition in a proportion between 0.5 and 10% by weight relative to the total weight of the composition.

4. Cosmetic or dermatological composition according to any one of the preceding claims, characterized in that the ratio between the surface-active agent and the dephasing agent is between 0.1/1 and 200/1.

5. Cosmetic or dermatological composition according to any one of the preceding claims, characterized in that the oily phase contains at least one oil taken from the group consisting of: liquid paraffin, isohexadecane, a silicone oil, a synthetic oil or a vegetable oil chosen from jojoba oil and safflower oil.

6. Cosmetic or dermatological composition according to Claim 5, characterized in that the synthetic oil is an alkyl palmitate or an alkyl adipate, the alkyl radical having from 2 to 10 carbon atoms.

7. Cosmetic or dermatological composition according to any one of the preceding claims, characterized in that the oily phase consists of at least one oil chosen from a cyclomethicone in a proportion of 1 to 80%, from 1 to 50% of isohexadecane, from 1 to 50% of octyl palmitate and of dioctyl adipate in a proportion of 1 to 50% by weight relative to the total weight of the oily phase.

8. Cosmetic or dermatological composition according to any one of the preceding claims, characterized in that it additionally contains, in at least one of the two phases, at least one conventional cosmetic adjuvant taken from the group consisting of: a perfume, a preserving agent, a dye, a softening agent, a buffer, a moistening agent and an electrolyte.

## Patentansprüche

1. Nichtschäumende, kosmetische oder dermatologische Zubereitung in zweiphasiger Form, die aus einer separaten wäßrigen und einer separaten Ölphase in einem Verhältnis von 30/70 bis 60/40 besteht, wobei mindestens eine dieser Phasen ein oberflächenaktives Mittel enthält, dadurch gekennzeichnet, daß sie in der wäßrigen Phase zusätzlich ein Phasentrennmittel in einer Menge von 0,025 bis 5 Gew.-%, wobei es sich bei dem Phasentrennmittel um ein Alkyldimethylbenzylammoniumchlorid der Formel 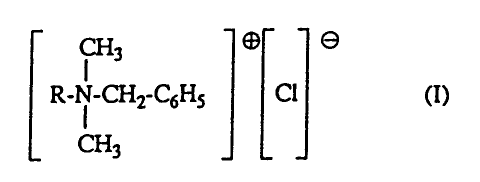 handelt, in der
R einen linearen gesättigten Alkylrest mit 12 bis 16 Kohlenstoffatomen darstellt, oder ein Gemisch von Alkyldimethylbenzylammoniumchloriden der Formel (1) enthält, und daß das oberflächenaktive Mittel in einer Menge von über 0,25 Gew.-%, bezogen auf das Gewicht der Zubereitung, vorhanden ist und anionischer, nichtionischer oder amphoterer Natur ist, wenn es in der wäßrigen Phase vorliegt, bzw. nichtionischer lipophiler Art, wenn es in der Ölphase ist.

2. Zubereitung gemäß Anspruch 1, dadurch gekennzeichnet, daß es sich bei dem Phasentrennmittel um eine Mischung aus etwa 65 Gew.-% Lauryldimethylbenzylammoniumchlorid, etwa 23 Gew.-% Myristyldimethylbenzylammoniumchlorid und etwa 8 Gew.-% Palmityldimethylbenzylammoniumchlorid handelt, wobei die Restmenge aus mindestens einem Alkyldimethylammoniumchlorid mit einem Alkylrest mit weniger als 12 oder mehr als 16 Kohlenstoffatomen besteht.

3. Kosmetische oder dermatologische Zubereitung gemäß einem der vorstehenden Ansprüche, dadurch gekennzeichnet, daß das oberflächenaktive Mittel in mindestens einer der beiden Phasen der Zubereitung in einer Menge von 0,5 bis 10 Gew.-%, bezogen auf das Gesamtgewicht der Zubereitung, vorhanden ist.

4. Kosmetische oder dermatologische Zubereitung gemäß einem der vorstehenden Ansprüche, dadurch gekennzeichnet, daß das Verhältnis von oberflächenaktivem Mittel zu Phasentrennmittel zwischen 0,1/1 und 200/1 liegt.

5. Kosmetische oder dermatologische Zubereitung gemäß einem der vorstehenden Ansprüche, dadurch gekennzeichnet, daß die Ölphase mindestens ein Öl, ausgewählt aus der aus Vaselinöl, Isohexadecan, einem Silikonöl, einem synthetischen oder einem unter Jojoba- und Safloröl ausgewählten pflanzlichen Öl bestehenden Gruppe, enthält.

6. Kosmetische oder dermatologische Zubereitung gemäß Anspruch 5, dadurch gekennzeichnet, daß es sich bei dem synthetischen Öl um ein Alkylpalmitat oder -adipat handelt, wobei der Alkylrest 2 bis 10 Kohlenstoffatome aufweist.

7. Kosmetische oder dermatologische Zubereitung gemäß einem der vorstehenden Ansprüche, dadurch gekennzeichnet, daß die Ölphase mindestens aus einem Öl besteht, das ausgewählt ist aus einem Cyclomethicon in einer Menge von 1 bis 80 %, Isohexadecan in einer Menge von 1 bis 50 %, 1 bis 50 % Octylpalmitat und Dioctyladipat in einer Menge von 1 bis 50 Gew.-%, bezogen auf das Gesamtgewicht der Ölphase.

8. Kosmetische oder dermatologische Zubereitung gemäß einem der vorstehenden Ansprüche, dadurch gekennzeichnet, daß sie außerdem in mindestens einer der beiden Phasen mindestens einen üblichen kosmetischen Hilfsstoff enthält, welcher aus der aus einem Parfüm, einem Konservierungsmittel, einem Farbstoff, einem Glättungsmittel, einem Füllstoff, einem Feuchthaltemittel und einem Elektrolyten (Puffer) ausgewählt ist.
